# EUROPÄISCHE PATENTSCHRIFT

(11) **EP 3 419 546 B1**
(45) Veröffentlichungstag und Bekanntmachung des Hinweises auf die Patenterteilung: **30.03.2022**
(21) Anmeldenummer: 17706464.9
(22) Anmeldetag: 21.02.2017
(51) Int. Cl.: A61B 50/30, A61L 2/07, A61L 2/26, A61B 50/00

(54) **STERILBEHÄLTERDECKEL MIT AUSSENABDECKUNG**
STERILE CONTAINER LID WITH OUTER COVER
COUVERCLE DE RÉCIPIENT STÉRILE COMPORTANT UN CACHE EXTÉRIEUR

(30) Priorität: 26.02.2016 DE 102016103401
(43) Veröffentlichungstag der Anmeldung: 02.01.2019
(73) Patentinhaber: Aesculap AG, 78532 Tuttlingen (DE)
(72) Erfinder: AICHER, Gerhard, 78600 Kolbingen (DE)
(74) Vertreter: Winter, Brandl - Partnerschaft mbB
(86) Internationale Anmeldenummer: PCT/EP2017/053914
(87) Internationale Veröffentlichungsnummer: WO 2017/144454

(56) Entgegenhaltungen:
- WO-A1-03/041604
- US-A1- 2005 004 551

## Beschreibung

Die Erfindung betrifft einen Deckel für einen Sterilbehälter mit einem Deckelbauteil, an dem zumindest ein, insbesondere perforierter, Gasaustauschabschnitt vorgesehen ist, zumindest einem Rahmen zur Aufnahme einer Filtereinrichtung, welcher an einer Deckelinnenseite an dem zumindest einen Gasaustauschabschnitt vorgesehen ist, und zumindest einer Außenabdeckung, welche den zumindest einen Gasaustauschabschnitt an einer Deckelaußenseite abdeckt.

Außerdem betrifft die Erfindung einen Sterilbehälter mit einem derartigen Deckel.

Klinische Instrumente, Gegenstände oder Ähnliches, welche beispielsweise mit Wunden oder offenen Körperstellen in Berührung kommen, müssen steril sein bzw. vor ihrem Einsatz am Menschen einer Sterilisation unterzogen werden. Darunter versteht man die Sicherstellung einer vollständigen Abwesenheit von Bakterien, Pilzen, Viren und Sporen bzw. das Abtöten aller Mikroorganismen, mit dem Ziel, eine absolute Keimfreiheit der entsprechenden Instrumente und Gegenstände zu erreichen. Die Instrumente und Gegenstände werden dabei in einen Sterilbehälter gegeben, in welchem die Sterilisation durchgeführt wird.

Als Sterilisationsverfahren wird insbesondere die Dampfsterilisation, auch Sterilisation durch feuchte Hitze genannt, eingesetzt, in welcher zunächst in einer Entlüftungsphase ein Vakuum in einem Sterilbehälter erzeugt wird und anschließend in einer Sterilisierphase Dampf mit hoher Hitze und hohem Druck in den Sterilbehälter gelangt und dadurch Mikroorganismen, welche sich auf den zu sterilisierenden Gegenständen befinden, zerstört werden. Daneben werden unter anderem auch chemische Sterilisationsverfahren und Sterilisation durch Strahlung eingesetzt.

Im Stand der Technik sind ein Deckel für einen Sterilbehälter, ein Sterilbehälter und eine Außenabdeckung für einen Sterilbehälterdeckel gemäß der Oberbegriffe der Ansprüche 1, 7 und 8 bekannt. Hierbei handelt es sich um einen Kunststoffdeckel mit integriertem, permanentem Keimrückhaltesystem, der für 5.000 Sterilisationszyklen validiert ist. Dieser weist ein Deckelbauteil aus Kunststoff, insbesondere Polyarylsulfon (PPSU) auf, welches an der Deckelinnenseite im Spritzgussverfahren angespritzte Zapfen aufweist, an welchen ein Rahmen zur Aufnahme einer Filtereinrichtung mittels Ultraschallschweißen anbringbar ist.

Eine Außenabdeckung aus Kunststoff, insbesondere aus Polyarylsulfon, deckt den perforierten Gasaustauschabschnitt zum Schutz der Filtereinrichtung vor mechanischen Einflüssen, beispielsweise während eines Transports oder einer Lagerung, ab. In dem bekannten Sterilbehälterdeckel wird die Außenabdeckung über an diese im Spritzgussverfahren angespritzte Klammern an dem Deckelbauteil befestigt.

Es hat sich jedoch in dem aus dem Stand der Technik bekannten Sterilbehälterdeckel herausgestellt, dass selbst Polyarylsulfon (PPSU) als Hochtemperatur- und Hochleistungskunststoff sehr alterungsanfällig, insbesondere nach mehr als 5.000 Sterilisationszyklen, ist. Es hat sich herausgestellt, dass Kunststoffe als Material für Sterilbehälterdeckel durch die Temperatur- und Druckbedingungen in der Dampfsterilisation oder durch die chemischen Stoffe in chemischen Sterilisationsverfahren angegriffen werden und es zu einer allmählichen Alterung, welche mit einer Freisetzung von Weichmachern und einer Rissbildung in dem Sterilbehälterdeckel bzw. in der Außenabdeckung verbunden ist, kommt.

Außerdem hat sich herausgestellt, dass eine Außenabdeckung aus Kunststoff, insbesondere Polyarylsulfon, mit zunehmender Alterung nicht mehr ausreichend schlag- und kratzfest ist und keinen ausreichenden Schutz vor mechanischen Einflüssen gewährleistet.

Aus diesen Gründen wird angestrebt, den Kunststoffdeckel aus dem Stand der Technik durch einen Metalldeckel zu ersetzen, welcher weniger alterungsanfällig ist und somit den Prozessbedingungen während einer Sterilisation besser standhält. Dabei hat sich jedoch das Problem ergeben, dass das Verbindungskonzept bzw. die Anbringung der an dem Deckelbauteil befestigten Bauteile wie des Rahmens und der Außenabdeckung nicht von einem Kunststoffdeckel auf einen Metalldeckel übertragbar ist. Insbesondere die Gestaltungsfreiheit im Spritzgießverfahren ermöglicht nämlich bei Kunststoffdeckeln bzw. -abdeckungen ein beliebiges Vorsehen bzw. Anspritzen von Zapfen oder Klammern, über welche die Verbindung der Einzelteile zueinander hergestellt werden kann.

Aus der WO 03/041604 A1 ist ein Deckel für einen Sterilbehälter gemäß dem Oberbegriff des Anspruchs 1 bekannt.

Der Stand der Technik hat somit den Nachteil, dass Kunststoffe als Werkstoffe für Sterilbehälterdeckel insbesondere aufgrund der Prozessbedingungen während der Sterilisation alterungsanfällig und somit rissanfällig sind und das in dem Kunststoffdeckel verwendete Verbindungskonzept nicht auf andere Materialien, insbesondere nicht auf Metalle, übertragbar ist.

Es ist also die Aufgabe der Erfindung, die Nachteile aus dem Stand der Technik zu vermeiden bzw. zu umgehen. Insbesondere soll eine geeignete Metallaußenabdeckung und ein Verbindungskonzept zum Anbringen bzw. Befestigen des Rahmens und der Außenabdeckung an einem Deckelbauteil aus Metall bereitgestellt werden. Dabei soll insbesondere die Befestigung der Außenabdeckung und des Rahmens an dem Deckelbauteil in einfacher Weise realisierbar sein und die Anzahl der verwendeten Verbindungselemente gering gehalten werden. Außerdem soll die Außenabdeckung in einfacher Weise herstellbar und an dem Deckelbauteil montierbar sein.

Diese Aufgabe wird durch einen Deckel für einen Sterilbehälter mit den Merkmalen des Anspruchs 1 und einen Sterilbehälter mit den Merkmalen des Anspruchs 7 gelöst. Vorteilhafte Weiterbildungen sind Gegenstand von Unteransprüchen.

Erfindungsgemäß wird ein Deckel für einen Sterilbehälter bereitgestellt, mit einem Deckelbauteil, insbesondere aus Metall, an dem zumindest ein, insbesondere perforierter, Gasaustauschabschnitt, vorgesehen ist, zumindest einem Rahmen zur Aufnahme einer Filtereinrichtung, welcher an einer Deckelinnenseite an dem zumindest einen Gasaustauschabschnitt vorgesehen ist, zumindest einer Außenabdeckung, insbesondere aus Metall, welche den zumindest einen Gasaustauschabschnitt an einer Deckelaußenseite abdeckt, und ferner mit zumindest einem an dem Deckelbauteil eingefügten/ angebrachten Verbindungselement/ Verbindungsteil, über welches an der Deckelaußenseite die Außenabdeckung und an der Deckelinnenseite der Rahmen befestigbar ist.

Somit ist über ein in das Deckelbauteil eingefügtes/ angebrachtes/ integriertes Verbindungselement bzw. Verbindungsteil/ -bauteil sowohl die Außenabdeckung zum Schutz der Filtereinrichtung und zur Abdeckung des Gasaustauschabschnitts als auch der Rahmen zur Aufnahme der Filtereinrichtung befestigt, so dass über ein Verbindungselement zwei Verbindungen, nämlich eine Verbindung an der Deckelinnenseite und eine Verbindung an der Deckelaußenseite eingegangen werden kann. Somit ist es möglich, die Anzahl der Verbindungselemente gering zu halten und auch bei Metallbauteilen eine geeignete Anbringung des Rahmens und der Außenabdeckung an das Deckelbauteil über das zwei Verbindungen eingehende Verbindungselement/ -teil zu gewährleisten.

Es ist dabei zweckmäßig, wenn das Verbindungselement ein bolzenartiges Verbindungselement ist und einen Befestigungsabschnitt aufweist, an dem das Verbindungselement kraftschlüssig, insbesondere durch Einpressen, an dem Deckelbauteil befestigt ist.

Somit wird eine kraft-/ bzw. reibschlüssige, das heißt spielfreie, feste Verbindung zwischen dem Befestigungsabschnitt des bolzenartigen Verbindungselements und dem Deckelbauteil, vorzugsweise durch Einpressen des Verbindungselements in eine Bohrung des Deckelbauteils, hergestellt. Insbesondere wird von dem Befestigungsabschnitt des Verbindungselements und der Bohrung des Deckelbauteils dabei eine Übermaßpassung bzw. Presspassung ausgebildet. Das bolzenartige Verbindungselement, insbesondere ein Bolzen, kann einen Anschlag an dem Befestigungsabschnitt aufweisen, über welchen der Einpressweg in axialer Richtung begrenzbar ist. Ferner kann das Verbindungselement sowohl von der Deckelinnenseite als auch von der Deckelaußenseite an das Deckelbauteil befestigt bzw. angebracht sein. Die Verbindung zwischen dem Deckelbauteil und dem Verbindungselement ist somit in einfacher Weise herstellbar und es sind keine zusätzlichen Befestigungsmittel wie Schrauben, Niete, etc. zur Befestigung des Verbindungselements an dem Deckelbauteil vonnöten, weshalb auch keine separaten Befestigungsmittel von der Deckelaußenseite oder der Deckelinnenseite sichtbar sind. Es wird somit eine geeignete Befestigung des Verbindungselements an dem Deckelbauteil ermöglicht.

Außerdem ist es von Vorteil, wenn das, insbesondere bolzenartige, Verbindungselement, welches vorzugsweise ein Bolzen ist, nach innen zu der Deckelinnenseite hin und nach außen zu der Deckelaußenseite hin vorsteht/ herausragt und einen inneren, ersten Verbindungsabschnitt/ Innenverbindungsabschnitt und einen äußeren, zweiten Verbindungsabschnitt/ Außenverbindungsabschnitt ausbildet.

Das Verbindungselement ist in vorteilhafter Weise ein Bolzen und nach Art eines Doppelbolzens ausgebildet. Dabei sind der innere, erste Verbindungsabschnitt des Verbindungselements zur Befestigung bzw. Anbringung des Rahmens und der äußere, zweite Verbindungsabschnitt zur Befestigung bzw. Anbringung der Außenabdeckung vorgesehen. Beide Verbindungsabschnitte können eine Begrenzung bzw. Beschränkung der axialen Einführung des Bolzens in die Außenabdeckung bzw. in den Rahmen, insbesondere in der Form eines Anschlags bzw. einer Durchmesservergrößerung aufweisen. Das Verbindungselement kann somit in anderen Worten zapfenartige und flanschartige Abschnitte umfassen. Dadurch kann eine definierte Position von sowohl Außenabdeckung zu Deckelbauteil als auch Rahmen zu Deckelbauteil gewährleistet werden. Die Ausbildung eines Innenverbindungsabschnitts und eines Außenverbindungsabschnitts hat allgemein den Vorteil, dass mit einem Verbindungselement, insbesondere mit einem Bolzen, zwei Bauteile an dem Deckelbauteil befestigt werden können, welche vorzugsweise dabei auch zueinander und zu dem Deckelbauteil positioniert werden. Es ist somit möglich, zwei Bauteile, nämlich den Rahmen und die Außenabdeckung verdreh- und verschiebefest an dem Verbindungselement und somit an dem Deckelbauteil zu befestigen.

Ein vorteilhaftes Ausführungsbeispiel ist dadurch gekennzeichnet, dass die Außenabdeckung an der Außenabdeckungsinnenseite zumindest eine Aufnahme zum kraftschlüssigen und/oder formschlüssigen Aufnehmen, insbesondere Klemmen, Einrasten oder Aufklipsen, des äußeren, zweiten Verbindungsabschnitts/ Außenverbindungsabschnitts des bolzenartigen Verbindungselements, insbesondere des Bolzens, aufweist.

Die Außenabdeckung kann somit erfindungsgemäß auf/in den vorstehenden/ herausstehenden Bolzen/ das bolzenartige Verbindungselement aufgesetzt/ aufgeschoben/ eingeführt/ aufgeklipst/ eingerastet/ geklemmt werden, so dass eine kraftschlüssige und/oder formschlüssige Verbindung zwischen dem Bolzen/ dem bolzenartigen Verbindungselement/ dem äußeren, zweiten Verbindungsabschnitt des bolzenartigen Verbindungselements und der Außenabdeckung entsteht. Vorzugsweise ist dabei erfindungsgemäß eine Federklemmscheibe zur kraftschlüssigen Aufnahme des Außenverbindungsabschnitts des bolzenartigen Verbindungselements vorgesehen. Es kann somit eine sichere Verbindung zwischen der Außenabdeckung und dem Deckelbauteil hergestellt werden, indem insbesondere ein vorstehender Bolzen in eine an der Außenabdeckungsinnenseite vorgesehene Federklemmscheibe eingeführt und geklemmt wird.

Dadurch, dass die Aufnahme an der Außenabdeckungsinnenseite vorgesehen ist, ist der Bolzen bzw. das Verbindungselement von der Außenabdeckungsaußenseite nicht sichtbar. Dies hat den Vorteil, dass keine von außen sichtbare Verbindung zwischen dem Deckelbauteil und der Außenabdeckung, wie dies beispielsweise bei Verschraubungen häufig der Fall ist, vorliegt. Des Weiteren ist es dadurch möglich, eine glatte Außenfläche vorzusehen, auf der es zu keinen Feuchtigkeitsansammlungen kommt.

Es ist somit erfindungsgemäß möglich, eine unsichtbare, einfache, dauerhafte und dauerfeste Verbindung zwischen der Außenabdeckung und dem Deckelbauteil, welche vorzugsweise über lediglich einem Klick herstellbar ist, bereitzustellen. Außenabdeckungsinnenseite und Außenabdeckungsaußenseite bilden zusammen die in den abhängigen Ansprüchen 8 bis 10 beanspruchte, Außenabdeckung, welche erfindungsgemäß vorzugsweise ein integrales, einstückiges und/oder einmaterialiges Bauteil ist. Es ist erfindungsgemäß jedoch auch denkbar, an der Außenabdeckungsinnenseite ein separates Klemmbauteil vorzusehen, welches, insbesondere stoffschlüssig, an die Außenabdeckung angebracht ist.

Ferner ist es zweckmäßig, wenn der Rahmen kraftschlüssig, insbesondere durch Einpressen, oder formschlüssig, insbesondere durch Aufklipsen oder Einrasten, an dem inneren, ersten Verbindungsabschnitt/ dem Innenverbindungsabschnitt des bolzenartigen Verbindungselements befestigbar ist.

Vorzugsweise wird der Rahmen dabei eingepresst, aufgeklipst oder eingerastet, wodurch eine dreh- und verschiebefeste Verbindung zwischen dem Rahmen und dem Verbindungselement entsteht. Somit ist über den Innenverbindungsabschnitt eine sichere, einfache, dauerhafte und/oder dauerfeste Verbindung zwischen dem Rahmen und dem bolzenartigen Verbindungselements/ dem Bolzen herstellbar.

In einer vorteilhaften Ausführungsform weist die in dem Rahmen aufgenommene Filtereinrichtung eine Halterung und ein Filterelement auf und ist die Filtereinrichtung von der Deckelinnenseite von einer Innenabdeckung abgedeckt, wobei die Halterung und/oder das Filterelement und/oder die Innenabdeckung an dem Innenverbindungsabschnitt des bolzenartigen Verbindungselements befestigbar ist.

Es ist somit erfindungsgemäß vorgesehen, dass zusätzlich zu dem Rahmen auch die Halterung und/oder das Filterelement, welches vorzugsweise eine Filtermembran aus Polytetrafluorethylen (PTFE) ist, und/oder die Innenabdeckung an dem inneren, ersten Verbindungsabschnitt des bolzenartigen Verbindungselements befestigbar sind. Dies hat den Vorteil, dass über ein Verbindungselement nicht nur zwei, sondern drei, vier oder sogar fünf Bauteile, nämlich die Außenabdeckung, der Rahmen, die Halterung, das Filterelement und die Innenabdeckung an dem Deckelbauteil befestigt werden können. Dadurch ist es möglich, die Anzahl der benötigten Befestigungsmittel/ Verbindungsmittel wie etwa Schrauben, Niete, Verschweißungen, etc. weiter zu reduzieren.

In vorteilhafter Weise ist das Deckelbauteil aus einem, insbesondere sterilisationsbeständigem, Leichtmetall, vorzugsweise Aluminium, ausgebildet und/oder ist die Außenabdeckung aus einem Metall, insbesondere einem sterilisationsbeständigem und/oder verschleißbeständigem und/oder hochsteifen und/oder hochfesten Metall, vorzugsweise Edelstahl, insbesondere nach Art eines Metallblechs/ Stahlblechs, ausgebildet.

Das Verwenden eines Leichtmetalls, insbesondere von Aluminium, hat den Vorteil, dass ein sterilsationsbeständiges und alterungsunanfälliges Material für das Deckelbauteil verwendet wird, welches gleichzeitig die Masse des Sterilbehälters nicht übermäßig, im Vergleich zu einem Kunststoffdeckel, erhöht. Außerdem werden durch das Verwenden eines Leichtmetalls als Deckelbauteil die mechanischen Eigenschaften des Deckels im Vergleich zu Kunststoff in großem Maße erhöht, so dass der Deckel vor äußeren mechanischen Einwirkungen besser geschützt ist.

Das Verwenden eines Metalls, vorzugsweise von Edelstahl, für die Außenabdeckung hat den Vorteil, dass ein äußerst schlagfestes, formbeständiges, kratzfestes, sterilisationsbeständiges und alterungsunanfälliges Material zum Einsatz kommt. Wenn das Metall nach Art eines Metallblechs ausgebildet ist, ist zudem eine einfache und kostengünstige Herstellung der Abdeckung, beispielsweise durch ein Schneidverfahren, insbesondere Laserschneiden, Wasserstrahlschneiden, Brennschneiden, oder durch Stanzen mit nachfolgender geeigneter Umformung oder Verschweißung gewährleistet. Zudem kann dem Sterilbehälterdeckel durch Verwenden von Aluminium für das Deckelbauteil und von Edelstahl für die Außenabdeckung in einfacher Weise ein hochwertigeres Aussehen/ äußeres Erscheinungsbild/ Design gegeben werden.

In einer vorteilhaften Ausführungsform sind zumindest zwei, vorzugsweise drei, vier, fünf oder mehr, weiter vorzugsweise vier, Aufnahmen an der Außenabdeckungsinnenseite zum Aufnehmen des Außenverbindungsabschnitts des bolzenartigen Verbindungselements vorgesehen, welche über die Außenabdeckungsinnenseite gleichmäßig verteilt, insbesondere gleichverteilt, sind.

Die Anzahl der Verbindungselemente entspricht dabei der Anzahl der Aufnahmen an der Außenabdeckungsinnenseite, so dass in jede vorgesehene Aufnahme ein äußerer, zweiter Verbindungsabschnitt eines bolzenartigen Verbindungselements/ eines Bolzens aufgenommen ist. Dies hat gleichzeitig zur Folge, dass auch an der Deckelinnenseite dieselbe Anzahl an inneren, ersten Verbindungsabschnitten vorgesehen ist. Durch die gleichmäßige Verteilung der Aufnahmen an der Außenabdeckungsinnenseite und somit die gleichmäßige Verteilung der bolzenartigen Verbindungselemente, vorzugsweise um den Umfang des Gasaustauschabschnitts herum, kann eine gleichmäßige und somit keine einseitige bzw. keine punktuelle Kraftübertragung bzw. Krafteinleitung zwischen dem Deckelbauteil/ dem bolzenartigem Verbindungselement und der Außenabdeckung auf der einen Seite bzw. dem Rahmen auf der anderen Seite gewährleistet werden. Dies hat den Vorteil, dass sowohl der Rahmen als auch die Außenabdeckung in einfacher Weise sicher fixiert bzw. befestigt werden können.

Die vorliegende Erfindung betrifft außerdem einen Sterilbehälter mit einem einen Behälterboden und Behälterwände umfassenden Aufnahmebehälter und mit einem vorstehend beschriebenen Deckel, insbesondere einem Deckel nach einem der Ansprüche 1 bis 6, zum Verschließen des Sterilbehälters.

Gemäß einem bevorzugten Ausführungsbeispiel weist die Außenabdeckung zumindest eine Aufnahme an einer Außenabdeckungsinnenseite zum kraftschlüssigen und/oder formschlüssigen Aufnehmen, insbesondere Klemmen, Einrasten oder Aufklipsen, eines bolzenartigen Verbindungselements, vorzugsweise eines Bolzens, auf.

Somit ist das bolzenartige Verbindungselement, vorzugsweise der Bolzen, welches von dem Deckelbauteil nach außen vorsteht, an der Außenabdeckungsinnenseite aufgenommen und kann in einfacher Weise befestigt werden. Damit wird ermöglicht, dass das Verbindungselement von der Außenabdeckungsaußenseite nicht sichtbar ist. Vorzugsweise erfolgt die Befestigung durch Klemmen, Einrasten oder Aufklipsen, so dass zur Befestigung der Außenabdeckung auf dem Deckelbauteil lediglich ein Einführen bzw. Aufsetzen der zumindest einen Aufnahme der Außenabdeckung auf das zumindest eine bolzenartige Verbindungselement vonnöten ist. Somit wird eine äußerst einfache Montage der Außenabdeckung auf dem Deckelbauteil ermöglicht.

In einer vorteilhaften Ausführungsform ist die Aufnahme an einem Vorsprung vorgesehen, an dem eine Klemmeinrichtung, insbesondere eine Federklemmscheibe, zum Klemmen des bolzenartigen Verbindungselements ausgebildet ist und/oder ist die Klemmeinrichtung durch Einführen des bolzenartigen Verbindungselements in einen Eingriffszustand überführbar.

Dabei ist der Vorsprung vorzugsweise derart ausgebildet, dass sich unter der Klemmeinrichtung bzw. zu der Außenabdeckungsaußenseite hin ein Hohlraum bzw. ein Aufnahmeraum für das bolzenartige Verbindungselement bzw. den Bolzen befindet, in welchen der Bolzen eingeführt werden kann. Der Vorsprung ist dabei derart ausgebildet, dass die Länge des vorstehenden Abschnitts größer oder gleich der Länge des Außenverbindungsabschnitts des Bolzens ist.

Wird der Bolzen in die Klemmeinrichtung, insbesondere die Federklemmscheibe eingeführt, geht die Federklemmscheibe von einem normalen, ungespannten Zustand in einen nach außen bzw. zu der Außenabdeckungsaußenseite hin um- bzw. aufgebogenen Zustand über, in welchem sie eine Federkraft auf den eingeführten Bolzen ausübt und diesen reib- bzw. kraftschlüssig fixiert, so dass eine Herausrutschen bzw. eine axiale Bewegung des Bolzens bzw. der Außenabdeckung verhindert wird. Vorzugsweise ist die Außenabdeckung nur durch große Krafteinwirkung wieder entfernbar. Es wird somit ein einfaches Einführen, eine sichere Befestigung bei gleichzeitigem Verhindern eines Herausrutschens erreicht. Somit kann der Kerngedanke der vorliegenden Erfindung, die Außenabdeckung in einfacher Weise an dem Deckelbauteil zu befestigen, gewährleistet werden. Zusätzlich zu der Federkraft kann auch ein Einrasten bzw. eine formschlüssige Aufnahme des Bolzens in der Außenabdeckung vorgesehen sein.

Es ist zweckmäßig, wenn die Außenabdeckung aus einem Blechteil gefertigt ist und der Vorsprung durch Umformen und/oder durch Aufklappen von zumindest einem Außenabdeckungseckabschnitt ausgebildet ist und/oder die Klemmeinrichtung, insbesondere die Federklemmscheibe, an dem Vorsprung durch Vorsehen einer Ausnehmung, insbesondere durch Stanzen, ausgebildet ist.

Die Fertigung bzw. die Herstellung der Außenabdeckung aus einem Blechteil hat dabei den Vorteil, dass sie einfach, kostengünstig und schnell durch Stanzen oder durch ein Schneidverfahren hergestellt werden kann. Dasselbe gilt für die Ausbildung des Vorsprungs durch Umformen eines Außenabdeckungseckabschnitts und die Ausbildung der Federklemmscheibe durch Vorsehen einer geeigneten Ausnehmung bereits vor dem Umformen. Somit kann die Gesamtfertigung der Außenabdeckung schnell, einfach und kostengünstig realisiert werden.

In vorteilhafter Weise ist der aufgeklappte Außenabdeckungseckabschnitt zumindest dreimal umgebogen und weist zumindest eine erste, eine zweite und eine dritte Biegekante auf und ist zwischen der zweiten und der dritten Biegekante ein im Wesentlichen zur Außenabdeckungsaußenseite paralleler Mittelabschnitt ausgebildet, welcher die Klemmeinrichtung aufweist und/oder ist ein Endabschnitt nach der dritten Biegekanten an der Außenabdeckung, vorzugsweise stoffschlüssig, befestigt.

Insbesondere die Befestigung eines Endabschnitts des Außenabdeckungseckabschnitts an der Außenabdeckung, vorzugsweise stoffschlüssig durch Schweißen, hat den Vorteil, dass der Vorsprung steif und stabil ausgebildet ist und die Verbindung von der Außenabdeckungsaußenseite nicht sichtbar ist. Ist zudem noch ein zu der Außenabdeckungsaußenseite paralleler Mittelabschnitt mit der Klemmeinrichtung vorgesehen, ist eine einfache Einführbarkeit eines Bolzens gewährleistet.

In einer vorteilhaften Ausführungsform ist ein separates Klemmbauteil an der Außenabdeckungsinnenseite, insbesondere stoffschlüssig, befestigt, welches die Aufnahme für ein bolzenartiges Verbindungselement, insbesondere für einen Bolzen aufweist.

Es kann somit erfindungsgemäß auch ein von der Außenabdeckung separates Klemmbauteil vorgesehen sein, welches stoffschlüssig an der Außenabdeckungsinnenseite angebracht ist. Dies hat den Vorteil, dass eine größere Gestaltungsfreiheit der Ausbildung des Vorsprungs gewährleistet ist im Vergleich zu einem umgebogenen Eckabschnitt.

Mit anderen Worten betrifft die Erfindung einen Doppelbolzen als Verbindungselement und eine äußere Abdeckung/ Außenabdeckung aus Stahlblech für den Containerdeckel/ Sterilbehälterdeckel PrimeLine aus Aluminium, wobei die Verbindung zwischen dem Containerdeckel und der Abdeckung nicht sichtbar ist.

Es werden daher zur Verbindung von Abdeckung zu Sterilbehälterdeckel keine Schrauben, Niete, etc. verwendet. Es soll eine hochwertige, schöne, stabile, kostengünstige und einfach zu montierende Abdeckung aus Metall bereitgestellt werden.

Erfindungsgemäß wird eine Feder-Klemmscheibe, welche an der Abdeckung vorgesehen ist, in einfacher Weise auf einen herausstehenden/ vorstehenden, in den Sterilbehälterdeckel eingepressten Bolzen eingerastet, wodurch eine sichere Verbindung zwischen der Abdeckung und dem Aluminiumdeckel ermöglicht wird. Dieser Bolzen ist ebenso nach innen vorstehend ausgebildet und hält an der Deckelinnenseite den Rahmen für einen Filter.

Es wird somit zum einen eine unsichtbare, einfache und dauerfeste Verbindung zwischen der Abdeckung und dem Deckel bereitgestellt, zum anderen können mit einem Bolzen bzw. Einpressbolzen, welcher sowohl nach innen als auch nach außen steht, zumindest zwei Verbindungen hergestellt werden.

Durch diesen Doppelbolzen kann somit sowohl die Abdeckung, welche aus einem Teil besteht, als auch der Rahmen an dem Deckel befestigt werden. Die Abdeckung kann vorzugsweise mit einem Klick auf dem Deckel montiert/ verrastet werden und deckt die Filterfläche des Containerdeckels ab. Vorzugsweise sind erfindungsgemäß vier Doppelbolzen vorgesehen, so dass sowohl der Innenrahmen/ Rahmen als auch die Abdeckung in einfacher Weise befestigt/fixiert werden können.

Die Erfindung wird mit Hilfe der nachfolgenden Figuren weiter erläutert. Es zeigen:
- Fig. 1: eine perspektivische Ansicht eines erfindungsgemäßen Sterilbehälters mit Deckel und Aufnahmebehälter,
- Fig. 2: eine Schnittansicht des erfindungsgemäßen Deckels, welche einen in ein Deckelbauteil eingepressten Bolzen zeigt, an dem auf einer Seite die erfindungsgemäße Außenabdeckung befestigt ist und an dem auf der anderen Seite ein Rahmen befestigt ist,
- Fig. 3: eine perspektivische Ansicht der Innenseite der erfindungsgemäßen Außenabdeckung,
- Fig. 4: eine perspektivische Ansicht der Außenseite der erfindungsgemäßen Außenabdeckung, und
- Fig. 5: eine perspektivische Ansicht der Innenseite des erfindungsgemäßen Deckels.

Die Figuren sind lediglich schematischer Natur und dienen ausschließlich dem Verständnis der Erfindung. Die gleichen Elemente sind mit denselben Bezugszeichen versehen. Die Merkmale der einzelnen Ausführungsbeispiele können untereinander ausgetauscht werden.

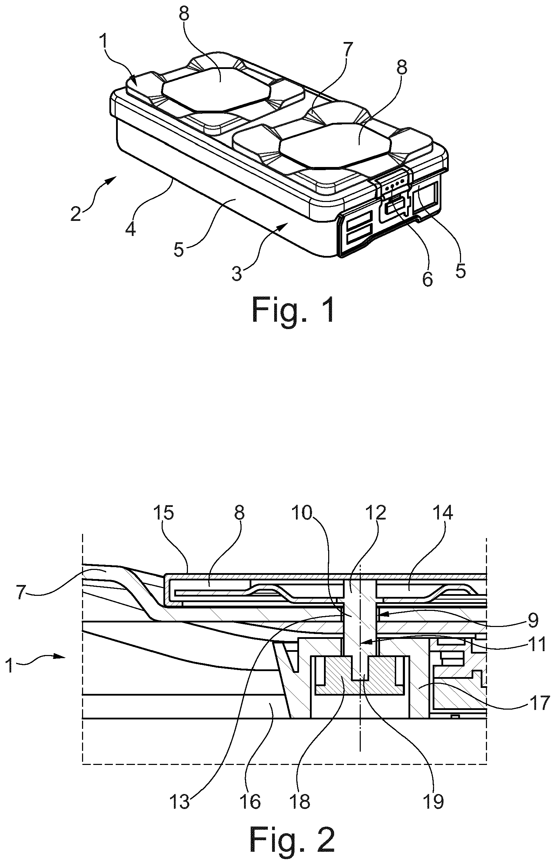

In Fig. 1 ist eine isometrische Ansicht eines erfindungsgemäßen Deckels 1 für einen Sterilbehälter 2 dargestellt, welcher einen Aufnahmebehälter 3 verschließt. Der Aufnahmebehälter 3 besteht dabei aus einem Behälterboden 4 und von diesem aufsteigende Behälterwände 5. Über einen Verschluss 6 werden der Aufnahmebehälter 3 und der Deckel 1 verschlossen. Der Deckel 1 weist ein Deckelbauteil 7 aus Aluminium auf, welches außen von zwei Außenabdeckungen 8 aus Stahlblech bedeckt ist. Unter den Außenabdeckungen 8 sind an dem Deckelbauteil 7 nicht dargestellte perforierte bzw. lochplattenartige Abschnitte vorgesehen. Von außen sind keine Schrauben, Nieten oder Ähnliches sichtbar, welche die Außenabdeckungen 8 an dem Deckelbauteil 7 befestigen.

Fig. 2 zeigt eine Schnittansicht des erfindungsgemäßen Deckels 1, welche einen in das Deckelbauteil 7 eingepressten Bolzen/ Verbindungselement 9 zeigt. Der Bolzen 9 weist einen Befestigungsabschnitt 10, einen Innenverbindungsabschnitt 11 und einen Außenverbindungsabschnitt 12 auf. Der Befestigungsabschnitt 10 des Bolzens 9 ist in eine Bohrung 13 des Deckelbauteils 7 eingepresst. Die Bohrung 13 und der Befestigungsabschnitt 10 bilden somit eine Presspassung aus. Der Bolzen 9 weist einen Flansch 14 auf, der die axiale Einführung bzw. Einpressung des Bolzens 9 von der Deckelaußenseite 15 an dem Deckelbauteil 7 begrenzt. Der Flansch 14 bildet somit einen Anschlag an dem Deckelbauteil 7 aus. An dem Außenverbindungsabschnitt 12 des Bolzens 9 ist die Außenabdeckung 8 befestigt. Der Flansch 14 bildet dabei ebenso einen Anschlag für die Außenabdeckung 8 aus. An der Deckelinnenseite 16 sind an dem Innenverbindungsabschnitt 11 des Bolzens sowohl ein Rahmen 17 als auch ein weiteres Bauteil, beispielsweise eine Filterhalterung 18, angebracht. Dabei wird zunächst der Rahmen 17 auf den Innenverbindungsabschnitt 11 des Bolzens 9 gepresst und anschließend das weitere Bauteil, beispielsweise die Filterhalterung 18 auf einen Zapfen 19 des Innenverbindungsabschnitts 11 aufgesteckt. Somit sind sowohl der Rahmen 17 als auch die Filterhalterung 18 an dem Innenverbindungsabschnitt 11 befestigt.

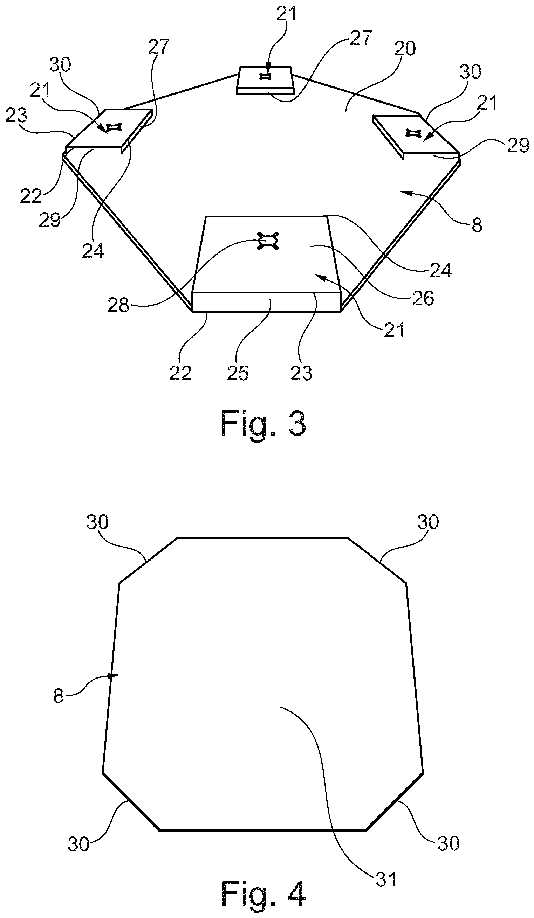

Fig. 3 zeigt eine isometrische Ansicht der Innenseite bzw. Außenabdeckungsinnenseite 20 der erfindungsgemäßen Außenabdeckung 8. An der Außenabdeckungsinnenseite 20 sind dabei vier Vorsprünge 21 vorgesehen. Die Vorsprünge 21 bestehen jeweils aus einer ersten Biegekante 22, einer zweiten Biegekante 23 und einer dritten Biegekante 24 bzw. aus einem Anfangsabschnitt 25 zwischen der ersten Biegekante 22 und der zweiten Biegekante 23, einem Mittelabschnitt 26 zwischen der zweiten Biegekante 23 und der dritten Biegekante 24 und einem Endabschnitt 27 nach der dritten Biegekante 24. An den Mittelabschnitten 26 ist jeweils mittig eine Aufnahme/ Federklemmscheibe/ Ausnehmung/ Klemmeinrichtung 28 vorgesehen. Der Anfangsabschnitt 25, der Mittelabschnitt 26 und der Endabschnitt 27 bzw. die erste Biegekante 22, die zweite Biegekante 23 und die dritte Biegekante 24 werden durch Umformen der Außenabdeckung 8, welche ein Blechteil ist, hergestellt. Die Endabschnitte 27 sind an der Außenabdeckung 8 durch Schweißen befestigt. Ein Hohlraum 29 ist von dem Anfangsabschnitt 25, dem Mittelabschnitt 26 und dem Endabschnitt 27 umgeben, in welchem ein Außenverbindungsabschnitt 12 eines Bolzens 9, welcher in die Federklemmscheibe/ Aufnahme/ Ausnehmung/ Klemmeinrichtung 28 eingeführt wird, aufgenommen werden kann. Die Vorsprünge 21 in Fig. 3 sind jeweils an Außenabdeckungseckabschnitten 30 vorgesehen. Die Vorsprünge 21 sind einstückig, einmaterialig und integral mit der Außenabdeckung 8 ausgebildet.

Fig. 4 zeigt eine isometrische Ansicht der Außenabdeckungsaußenseite 31 der erfindungsgemäßen Außenabdeckung 8. Die Außenabdeckungsaußenseite 31 stellt dabei eine glatte, ebene Fläche dar. Es sind von der Außenabdeckungsaußenseite 31 keine Verbindungselement sichtbar. Die Außenabdeckungseckabschnitte 30 sind eingezogen ausgebildet. Aufgrund der eingezogenen Außenabdeckungseckabschnitte 30 ist die Außenabdeckung 8 achteckig ausgebildet.

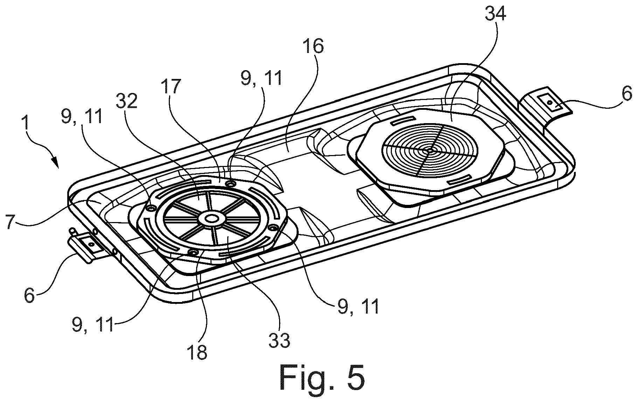

Fig. 5 zeigt eine isometrische Ansicht einer Deckelinnenseite 16 des erfindungsgemäßen Deckels 1. An dem Deckelbauteil 7 ist links an der Deckelinnenseite 16 der Rahmen 17 vorgesehen, in welchem eine Filtereinrichtung 32 aufgenommen ist. Die Filtereinrichtung 32 besteht aus der Filterhalterung 18 und dem Filterelement/ der Filtermembran 32. An vier Innenverbindungsabschnitten 11 der Bolzen 9 ist der Rahmen 17 an dem Deckelbauteil 7 fixiert. Rechts ist in Fig. 5 die links gezeigte Anordnung von einer Innenabdeckung 34 bedeckt dargestellt.

### Bezugszeichenliste

- 1: Deckel
- 2: Sterilbehälter
- 3: Aufnahmebehälter
- 4: Behälterboden
- 5: Behälterwand
- 6: Verschluss
- 7: Deckelbauteil
- 8: Außenabdeckung
- 9: Bolzen/ Verbindungselement
- 10: Befestigungsabschnitt
- 11: Innenverbindungsabschnitt
- 12: Außenverbindungsabschnitt
- 13: Bohrung
- 14: Flansch
- 15: Deckelaußenseite
- 16: Deckelinnenseite
- 17: Rahmen
- 18: Filterhalterung
- 19: Zapfen
- 20: Außenabdeckungsinnenseite
- 21: Vorsprung
- 22: Erste Biegekante
- 23: Zweite Biegekante
- 24: Dritte Biegekante
- 25: Anfangsabschnitt
- 26: Mittelabschnitt
- 27: Endabschnitt
- 28: Federklemmscheibe/ Aufnahme/ Ausnehmung/ Klemmeinrichtung
- 29: Hohlraum
- 30: Außenabdeckungseckabschnitt
- 31: Außenabdeckungsaußenseite
- 32: Filtereinrichtung
- 33: Filterelement/ Filtermembran
- 34: Innenabdeckung

## Patentansprüche

1. Deckel (1) für einen Sterilbehälter (2), mit
einem Deckelbauteil (7), insbesondere aus Metall, an dem zumindest ein, insbesondere perforierter, Gasaustauschabschnitt vorgesehen ist;
zumindest einem Rahmen (17) zur Aufnahme einer Filtereinrichtung (32), welcher an einer Deckelinnenseite (16) an dem zumindest einen Gasaustauschabschnitt vorgesehen ist; und
zumindest einer Außenabdeckung (8), insbesondere aus Metall, welche den zumindest einen Gasaustauschabschnitt an einer Deckelaußenseite (15) abdeckt,
**gekennzeichnet durch** zumindest ein an dem Deckelbauteil (7) eingefügtes Verbindungselement (9), über welches an der Deckelaußenseite (15) die Außenabdeckung (8) und an der Deckelinnenseite (16) der Rahmen (17) befestigbar ist.

2. Deckel (1) nach Anspruch 1, **dadurch gekennzeichnet, dass**
das Verbindungselement (9) ein bolzenartiges Verbindungselement (9) ist und einen Befestigungsabschnitt (10) aufweist, an dem das Verbindungselement (9) kraftschlüssig, insbesondere durch Einpressen, an dem Deckelbauteil (7) befestigt ist.

3. Deckel (1) nach Anspruch 1 oder 2, **dadurch gekennzeichnet, dass**
das Verbindungselement (9) nach innen zu der Deckelinnenseite (16) hin und nach außen zu der Deckelaußenseite (15) hin vorsteht und einen inneren, ersten Verbindungsabschnitt (11) und einen äußeren, zweiten Verbindungsabschnitt (12) ausbildet.

4. Deckel (1) nach Anspruch 3, **dadurch gekennzeichnet, dass**
die Außenabdeckung (8) an der Außenabdeckungsinnenseite (20) zumindest eine Aufnahme (28) zum kraftschlüssigen und/oder formschlüssigen Aufnehmen, insbesondere Klemmen, Einrasten oder Aufklipsen, des äußeren, zweiten Verbindungsabschnitts (12) des bolzenartigen Verbindungselements (9) aufweist und/oder
der Rahmen (17) kraftschlüssig, insbesondere durch Einpressen, und/oder formschlüssig, insbesondere durch Aufklipsen oder Einrasten, an dem inneren, ersten Verbindungsabschnitt (11) des bolzenartigen Verbindungselements (9) befestigbar ist.

5. Deckel (1) nach Anspruch 3 oder 4, **dadurch gekennzeichnet, dass**
die in dem Rahmen (17) aufgenommen Filtereinrichtung (32) eine Halterung (18) und ein Filterelement (33) aufweist und die Filtereinrichtung (32) von der Deckelinnenseite (16) von einer Innenabdeckung (34) abgedeckt ist, wobei die Halterung (18) und/oder das Filterelement (33) und/oder die Innenabdeckung (34) an dem inneren, ersten Verbindungsabschnitt (11) des bolzenartigen Verbindungselements (9) befestigbar ist.

6. Deckel (1) nach einem der vorhergehenden Ansprüche, **dadurch**
**gekennzeichnet, dass**
das Deckelbauteil (7) aus einem, insbesondere sterilisationsbeständigem, Leichtmetall, vorzugsweise Aluminium, ausgebildet ist und/oder
die Außenabdeckung (8) aus einem Metall, insbesondere einem sterilisationsbeständigem und/oder verschleißbeständigem und/oder hochsteifen und/oder hochfesten Metall, vorzugsweise Edelstahl, insbesondere nach Art eines Metallblechs, ausgebildet ist.

7. Sterilbehälter (2) mit einem einen Behälterboden (4) und Behälterwände (5) umfassenden Aufnahmebehälter (3), **gekennzeichnet durch** einen Deckel (1) nach einem der vorhergehenden Ansprüche zum Verschließen des Aufnahmebehälters (3).

8. Deckel (1) nach Anspruch 1, **dadurch gekennzeichnet, dass**
die Außenabdeckung (8) zumindest eine Aufnahme (28) an einer Außenabdeckungsinnenseite (20) zum kraftschlüssigen und/oder formschlüssigen Aufnehmen, insbesondere Klemmen, Einrasten oder Aufklipsen, eines bolzenartigen Verbindungselements (9) aufweist.

9. Deckel (1) nach Anspruch 8, **dadurch gekennzeichnet, dass**
die Aufnahme (28) an einem Vorsprung (21) vorgesehen ist, an dem eine Klemmeinrichtung (28), insbesondere eine Federklemmscheibe (28), zum Klemmen des bolzenartigen Verbindungselements (9) ausgebildet ist und/oder die Klemmeinrichtung (28) durch Einführen des bolzenartigen Verbindungselements (9) in einen Eingriffszustand überführbar ist.

10. Deckel (1) nach Anspruch 9, **dadurch gekennzeichnet, dass**
diese aus einem Blechteil gefertigt ist und der Vorsprung (21) durch Umformen und/oder durch Aufklappen von zumindest einem Außenabdeckungseckabschnitt (30) ausgebildet ist und/oder die Klemmeinrichtung (28), insbesondere die Federklemmscheibe (28), an dem Vorsprung (21) durch Vorsehen einer Ausnehmung (28), insbesondere durch Stanzen, ausgebildet ist.

## Claims

1. Lid (1) for a sterile container (2), comprising:
a lid component (7), in particular made of metal, which is provided with at least one gas exchange section, in particular a perforated one;
at least one frame (17) for receiving a filter device (32) provided on a lid inner side (16) at the at laest one gas exchange section; and
at least one outer cover (8), particularly made of metal, covering the at least one gas exchange section on a lid outer side (15),
**characterized by** at least one connection element (9) inserted in the lid component (7) by way of which the outer cover (8) is attachable on the lid outer side (15) and the frame (17) is attachable on the lid inner side (16).

2. The lid (1) according to claim 1, **characterized in that**
the connection element (9) is a bolt-like connection element (9) and has a fastening section (10) at which the connection element (9) is fastened to the lid component (7) in a force-locking manner, in particular by press-fitting.

3. The lid (1) according to claim 1 or 2, **characterized in that**
the connection element (9) projects inwardly towards the lid inner side (16) and outwardly towards the lid outer side (15) and forms an inner first connecting portion (11) and an outer second connecting portion (12).

4. The lid (1) according to claim 3, **characterized in that**
the outer cover (8) has at least one receptacle (28) on the outer cover inner side (20) for force-locking or form-fitting reception, in particular by clamping, latching and clipping on, of the outer second connecting portion (12) of the bolt-like connection element (9) and/or
the frame (17) can be fastened in a force-locking manner, in particular by press-fitting, and/or in a form-fitting manner, in particular by clipping on or latching, to the inner first connecting portion (11) of the bolt-like connection element (9).

5. The lid (1) according to claim 3 or 4, **characterized in that**
the filter device (32) received in the frame (17) has a holder (18) and a filter element (33) and the filter device (32) is covered by an inner cover (34) from the lid inner side (16), the holder (18) and/or the filter element (33) and/or the inner cover (34) being attachable to the inner first connecting portion (11) of the bolt-like connection element (9).

6. The lid (1) according to any of the preceding claims, **characterized in that**
the lid component (7) is made of a sterilization-resistant, light metal, preferably aluminium, and/or
the outer cover (8) is made of a metal, in particular a sterilization-resistant and/or wear-resistant metal and/or a metal of high-modulus strength and/or high-strength, preferably stainless steel, particularly in the style of a sheet metal.

7. A sterile container (2) with a receiving container (3) comprising a container base (4) and container walls (5), **characterized by** a lid (1) according to one of the preceding claims for closing the receiving container (3).

8. The lid (1) according to claim 1, **characterized in that**
the outer cover (8) has at least one receptacle (28) on an outer cover inner side (20) for force-locking and/or form-fitting reception, in particular by clamping, latching or clipping on, a bolt-like connection element (9).

9. The lid (1) according to claim 8, **characterized in that**
the receptacle (28) is provided on a projection (21) on which a clamping device (28), in particular a spring clamping disk (28), is formed for clamping the bolt-like connection element (9), and/or the clamping device (28) is transferred into a state of engagement by inserting the bolt-like connection element (9).

10. The lid (1) according to claim 9, **characterized in that**
the same is manufactured from a sheet metal part and the projection (21) is formed by forming and/or folding at least one outer cover corner portion (30) and the clamping device (28), in particular the spring clamping disk (28), is formed on the projection (21) by providing a recess (28) formed in particular by way of punching.

## Revendications

1. Couvercle (1) pour un contenant stérile (2), avec
un élément de couvercle (7), en particulier en métal, sur lequel est prévue au moins une section d'échange gazeux, en particulier perforée ;
au moins un cadre (17) pour la réception d'un système de filtre (32), lequel est prévu sur une face intérieure de couvercle (16) sur la au moins une section d'échange gazeux ; et
au moins un cache extérieur (8), en particulier en métal, lequel recouvre la au moins une section d'échange gazeux sur une face extérieure de couvercle (15),
**caractérisé par** au moins un élément de liaison (9) encastré sur l'élément de couvercle (7), par l'intermédiaire duquel le cache extérieur (8) peut être fixé sur la face extérieure de couvercle (15) et le cadre (17) sur la face intérieure de couvercle (16).

2. Couvercle (1) selon la revendication 1, **caractérisé en ce que**
l'élément de liaison (9) est un élément de liaison (9) du type axe et présente une section de fixation (10), sur laquelle l'élément de liaison (9) est fixé à force, en particulier par enfoncement, sur l'élément de couvercle (7).

3. Couvercle (1) selon la revendication 1 ou 2, **caractérisé en ce que**
l'élément de liaison (9) fait saillie vers l'intérieur en direction de la face intérieure de couvercle (16) et vers l'extérieur en direction de la face extérieure de couvercle (15) et réalise une première section de liaison intérieure (11) et une deuxième section de liaison extérieure (12).

4. Couvercle (1) selon la revendication 3, **caractérisé en ce que**
le cache extérieur (8) présente sur la face intérieure de cache extérieur (20) au moins un logement (28) pour la réception à force et/ou par coopération de formes, en particulier serrage, encliquetage ou clipsage, de la deuxième section de liaison extérieure (12) de l'élément de liaison (9) du type axe
et/ou
le cadre (17) peut être fixé à force, en particulier par enfoncement, et/ou par coopération de formes, en particulier par clipsage ou encliquetage, sur la première section de liaison intérieure (11) de l'élément de liaison (9) du type axe.

5. Couvercle (1) selon la revendication 3 ou 4, **caractérisé en ce que**
le système de filtre (32) reçu dans le cadre (17) présente un élément de retenue (18) et un élément de filtre (33) et le système de filtre (32) de la face intérieure de couvercle (16) est recouvert par un cache intérieur (34), dans lequel l'élément de retenue (18) et/ou l'élément de filtre (33) et/ou le cache intérieur (34) peut être fixé sur la première section de liaison intérieure (11) de l'élément de liaison (9) du type axe.

6. Couvercle (1) selon l'une quelconque des revendications précédentes, **caractérisé en ce que**
l'élément de couvercle (7) est réalisé à partir d'un métal léger, en particulier résistant à la stérilisation, de préférence l'aluminium et/ou
le cache extérieur (8) est réalisé à partir d'un métal, en particulier d'un métal résistant à la stérilisation et/ou résistant à l'usure et/ou hautement rigide et/ou à haute résistance, de préférence l'acier inoxydable, en particulier à la façon d'une tôle métallique.

7. Contenant stérile (2) avec un contenant de réception (3) comprenant un fond de contenant (4) et des parois de contenant (5), **caractérisé par** un couvercle (1) selon l'une quelconque des revendications précédentes pour la fermeture du contenant de réception (3).

8. Couvercle (1) selon la revendication 1, **caractérisé en ce que**
le cache extérieur (8) présente au moins un logement (28) sur une face intérieure de cache extérieur (20) pour la réception à force et/ou par coopération de formes, en particulier serrage, encliquetage ou clipsage, d'un élément de liaison (9) du type axe.

9. Couvercle (1) selon la revendication 8, **caractérisé en ce que**
le logement (28) est prévu sur une partie saillante (21), sur laquelle est réalisé un système de serrage (28), en particulier un disque de serrage à ressort (28), pour le serrage de l'élément de liaison (9) du type axe et/ou le système de serrage (28) peut être amené dans un état de mise en prise par introduction de l'élément de liaison (9) du type axe.

10. Couvercle (1) selon la revendication 9, **caractérisé en ce que**
celui-ci est fabriqué à partir d'une pièce de tôle et la partie saillante (21) est réalisée par formage et/ou par dépliage d'au moins une section de coin de cache extérieur (30) et/ou le système de serrage (28), en particulier le disque de serrage à ressort (28), est réalisé sur la partie saillante (21) par prévision d'un évidement (28), en particulier par estampage.
